# EUROPEAN PATENT SPECIFICATION

(11) **EP 0 973 516 B1**
(45) Date of publication and mention of the grant of the patent: **23.06.2004**
(21) Application number: 98913904.3
(22) Date of filing: 25.03.1998
(51) Int. Cl.: A61K 31/35, A61P 25/22

(54) **USE OF HALO AND/OR NITRO-SUBSTITUTED FLAVONOIDS AS ANXYOLITICS**
VERWENDUNG VON HALO UND/ODER NITRO-SUBSTITUIERTEN FLAVONOIDEN ALS ANGSTLÖSER
UTILISATION DE FLAVONOIDES HALO ET/OU NITRO-SUBSTITUES EN TANT QU'ANXYOLITIQUES

(30) Priority: 07.04.1997 GB 9707013
(43) Date of publication of application: 26.01.2000
(73) Proprietor: University of Strathclyde, Glasgow G1 1YQ, Scotland (GB)
(72) Inventor: PALADINI, Alejandro, Constantino, Junin 956 1113 Buenos Aires (AR); MEDINA, Jorge, Horacio Instituto Biolúgico Celular, Paraguay 2155 1121 Buenos Aires (AR)
(74) Representative: MacDougall, Donald Carmichael
(86) International application number: PCT/GB1998/000906
(87) International publication number: WO 1998/044920

(56) References cited:
- WO-A-95/05169
- WO-A-97/14414
- MARDER M ET AL: "6,3'-DINITROFLAVONE, A NOVEL HIGH AFFINITY LIGAND FOR THE BENZODIAZEPINE RECEPTOR WITH POTENT ANXIOLYTIC PROPERTIES" BIOORGANIC & MEDICINAL CHEMISTRY LETTERS, vol. 5, no. 22, 1995, pages 2717-2720, XP000614386
- MARDER ET AL.: "6-Bromoflavone, a High Affinity Ligand for the Central Benzodiazepine Receptor Is a Member of a Family of Active Flavonoids" BIOCHEM. BIOPHYS. RES. COMM., vol. 223, no. 2, 1996, pages 384-389, XP002074118
- SALGUEIRO ET AL.: "Anxiolytic Natural and Synthetic Flavonoid Ligands on the Central Benzodiazepine Receptor Have No Effect on Memory Tasks in Rats" PHARMACOL. BIOCHEM. BEHAV., vol. 58, no. 4, 1997, pages 887-891, XP002074119
- WOLFMAN ET AL.: "Anxioselective properties of 6,3'-dinitroflavone, a high affinity benzodiazepine receptor ligand" EUR. J. PHARMACOL., vol. 318, no. 1, 1996, pages 23-30, XP002074120
- VIOLA ET AL.: "6-BROMO-3'-NITROFLAVONE, A NEW HIGH AFFINITY BENZODIAZEPINE RECEPTOR AGONIST RECOGNIZES TWO POPULATIONS OF CEREBRAL CORTICAL BINDING SITES" BIOORG. MED. CHEM. LETT., vol. 7, no. 3, 1997, pages 373-378, XP002074121
- CUNNINGHAM ET AL.: "Synthesis and biological evaluaation of aseries of flavones designed as inhibitors of protein tyrosine kinases" ANTI-CANCER DRUG DESIGN, vol. 7, no. 5, 1992, pages 365-384, XP002074122
- MARDER ET AL.: "SYNTHESIS OF HALOGENATED/NITRATED FLAVONE DERIVATIVES AND EVALUATION OF THEIR AFFINITY FOR THE CENTRAL BENZODIAZEPINE RECEPTOR" BIOORG. MED. CHEM. LETT., vol. 7, no. 15, 1997, pages 2003-2008, XP002074123

## Description

### Field of Invention

The present invention relates to flavonoids which have been found to have anxiolytic properties (i.e. anxiety reducing) without corresponding depression of the central nervous system which is commonly also found in known sedatives such as benzodiazepines. In particular, the present invention relates to flavonoids comprising nitro and/or halo groups located on the phenyl ring and/or flavone nucleus and analogues thereof.

### Background

Co-pending patent application WO 95/05169 (Strathclyde University) relates to flavonoids and their use in methods of treating anxiety in patients. The flavonoids of WO 95/05169 are described as having anxiolytic properties without associated depression of the central nervous system (e.g. sedative and muscle relaxant effects) commonly found with benzodiazepines. The compounds of WO 95/05169 fall under a general formula: 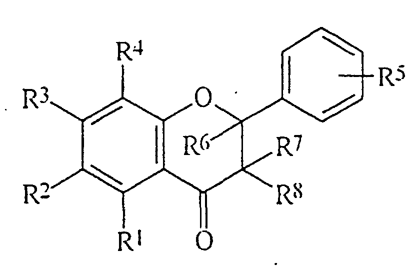 wherein
R¹, R², R³ and R⁴, R⁵ and R⁸ are independently selected from H, OH, R, NO₂, halo, OR, NH₂, NHR, NR₂, COOR, COOH, CN, or a sugar group;
R⁶ and R⁷ are both H, or R⁶ and R⁷ together form a single bond;
R is C₁₋₆ alkyl or alkenyl; or dimers thereof.

Preferred compounds of WO 95/05169 are described as halo derivatives, in particular where R⁵ is halo at the 2' position of the above general formula.

It has now been found that certain compounds falling within the generic formula of WO 95/05169 exhibit unexpectedly good anxiolytic activity without associated depression of the central nervous system (e.g. sedative and muscle relaxant effects) commonly found with benzodiazepines, when the flavone nucleus and/or phenyl ring is nitrated and/or halogenated. Thus, patients may be treated for anxiety without inducing sedative or myorelaxant side-effects.

It has also been found that compounds of the present invention display a substantially reduced or no anti-convulsant effect, and that memory is apparently not adversely affected, side-effects commonly found with benzodiazepines.

### Statement of Invention

The present invention relates to a method of treating anxiety in a patient with an administrable effective non-toxic amount of a flavonoid of general Formula (I): 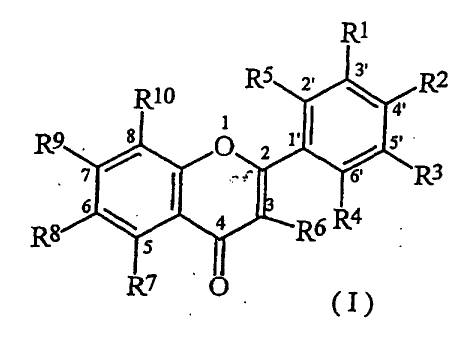 for the preparation of a medicament for the treatment of anxiety in a patient, wherein
R¹, R², R³, R⁴ and R⁵ are independently selected from Cl, Br, F and H;
R⁶ is H;
R⁷, R⁹ and R¹⁰ are independently selected from H, -OH, -R, -NO₂, Br, Cl, F, -OR, -NH₂, -NHR, -NR₂, -COOR, -COOH, -CN or a sugar group;
R⁸ is selected from H, NO₂, Br, Cl, and F;
R is C₁-C₆ alkyl or alkenyl;
with the provision that when R¹ is H, R⁸ is NO₂, when R¹ is selected from Cl, Br and F, R⁸ is selected from H, NO₂, Br, Cl and F, and at least one of R¹ to R⁵ or R⁶ to R¹⁰ is selected from the group consisting of F, Cl and Br;
or with an administrable effective non-toxic amount of a bi-flavonoid which is a dimer of a compound of general Formula (I) and wherein R¹ to R¹⁰ and R have the meanings given for general Formula (I).

The sugar group may be any of the known sugars, including monosaccharides, disaccharides and polysaccharides; and may in particular be glycosyl, galactopyranosyl or marinopyranosyl.

Preferred compounds pharmaceutical formulation comprise a flavonoid of Formula I for use for the treatment of anxiety in a patient include compounds wherein
R¹, R², R³, R⁴ and R⁵ are independently selected from Cl, Br, F and H;
R⁶ is H;
R⁷, R⁹ and R¹⁰ are independently selected from H, -OH, -R, -NO₂, Br, Cl, F, -OR, -NH₂, -NHR, -NR₂, -COOR, -COOH, -CN or a sugar group;
R⁸ is selected from H, NO₂, Br, Cl, and F;
R is C₁₋C₆ alkyl or alkenyl;
with the proviso that when R¹ is H, R⁸ is NO₂, when
R¹ is selected from Cl, Br and F, R⁸ is selected from H, NO₂, Br, Cl and F, and at least one of R¹ to R⁵ or R⁶ to R¹⁰ is selected from the group consisting of F, Cl and Br.

A formulation according to claim 14 wherein
R¹ is selected from H, Cl and Br;
R² to R⁷ inclusive, R⁹ and R¹⁰ are all H;
R⁸ is selected from H, NO₂, Br, Cl and F.

A formulation according to claim 14 or claim 15 wherein
R¹ is selected from H and Br;
R² to R⁷ inclusive, R⁹ and R¹⁰ are all H; and
R⁸ is selected from Br, NO₂, and H.

A formulation according to any one of claims 14 to 16 wherein
R¹ is Br;
R² to R⁷ inclusive, R⁹ and R¹⁰ are all H; and
R⁸ is selected from Br and NO₂.

Example of preferred compounds of formula I are :
3' chlorof lavone 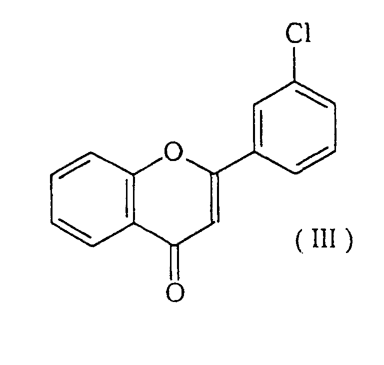
3' bromoflavone 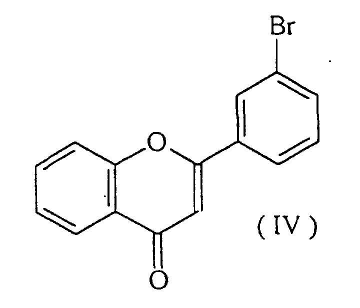
6,3'- dibromoflavone 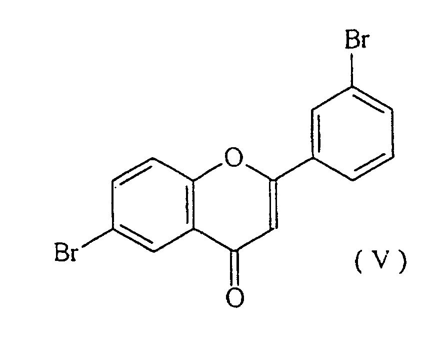
6, nitro, 3'bromoflavone

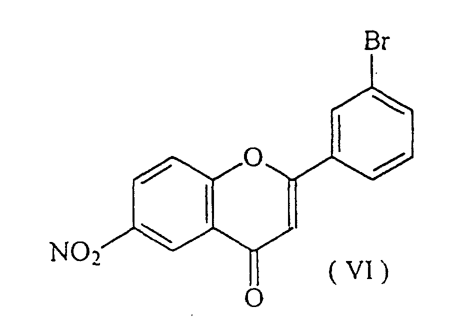

Especially preferred are compounds (V) and (VI) above.

Compounds wherein R⁶ and R⁷ together form a single bond are flavone derivatives.

The bi-flavonoid is a dimer of two covalently bonded moieties which are each of general Formula (I) as set out above. Bonding between the two moieties generally occurs at the 3"-position of one moiety and the 8-position of the other moiety. The preferred bi-flavonoid has general Formula (VII) wherein R¹ to R¹⁰ and R have the same meanings as for general Formula (I):

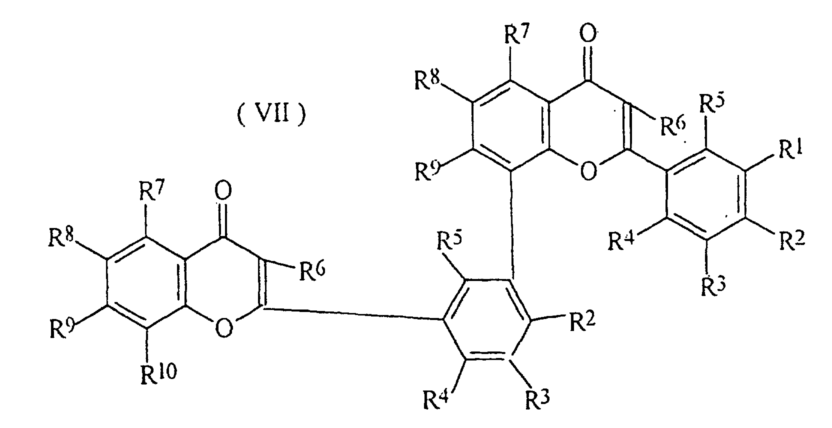

Preferred compounds of general Formula (VII) for use in a method of treating anxiety in a patient include those wherein at least R¹ in each of the dimer moieties of general Formula (I) is selected from H, Cl, and Br; R⁸ is selected from Cl, Br, F, H and NO₂; and all other R functional groups are as defined for Formula (I) with the proviso that when R¹ is H, R⁸ is NO₂ and when R¹ is selected from Cl, Br and F, R⁸ is selected from H, NO₂ Br, Cl, and F.

The compounds of general Formula (VII) wherein the compounds are 3' halo (ie Br, Cl or F) containing compounds; R⁸ is selected from NO₂, Cl and Br; and all other R functional groups are hydrogen are more preferred.

Pharmaceutical formulations include at least one compound of general Formula (I) and/or (VII) together with at least one pharmaceutically acceptable carrier or excipient. Naturally, the skilled addressee will appreciate that compounds of the invention employed in pharmaceutical formulations of the invention possess R groups R¹ to R¹⁰ and R as defined herein. Each carrier must be "pharmaceutically acceptable" in the sense of being compatible with the other ingredients of the formulations and not injurious to the patient.

It should be understood that the flavonoid compounds of the present invention can be administered in the form of pharmaceutically acceptable salts or esters thereof. Salts are usually acid addition salts (e.g. with hydrohalogen acids) or acceptable metal salts (e.g. Na, Ca, Mg).

Formulations include those adapted for oral, rectal, nasal, vaginal and parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention adapted for oral administration may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethylcellulose), lubricant, inert diluent, preservative, disintegrant (e.g. sodium starch glycolate, cross-linked povidone, cross-linked sodium carboxymethylcellulose) active-surface or dispersing agent. Moulded tablets may be made by moulding in a suitable machine a mixture of the powdered compound moistened with an inert liquid diluent. The tablets may optionally be coated or scored and may be,formulated so as to provide controlled release of the active ingredient therein using, for example, hydroxypropylmethylcellulose in varying proportions to provide the desired release profile.

Formulations for rectal administration may be presented as a suppository with a suitable base comprising for example cocoa butter or a salicylate.

Formulations for vaginal administration may be presented as pessaries, tampons, creams, gels, pastes, foams or spray formulations containing in addition to the active ingredient such carriers as are known in the art to be appropriate.

Formulations are parenteral administration include aqueous and non-aqueous. isotonic sterile injection solutions which may contain anti-oxidants, buffers, bacteriostatis and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze dried (lyophilized) condition requiring only the addition of the sterile carrier, for example water for injections, immediately prior to use. Extemporaneous injection solutions and suspensions may be prepared from sterile powders, granules and tablets of the kind previously described.

The dose will depend on a number of factors known to the skilled physician including the severity of the conditions, the identity of the recipient; and also the efficacy and toxicity of the particular compound of general Formula (I) which is being administered. Generally doses in the range 0.1-100 mg/kg body weight may be used, particularly 1-10 mg/kg. The frequency of administration will vary depending on the rate of metabolism or excretion of the administered compound, but may be repeated daily, optionally as two or more sub-doses. Unit doses of 20 to 500 mg, preferably 100 to 400 mg may be used.

As a further aspect of the present invention there is provided a compound of general Formula (I): 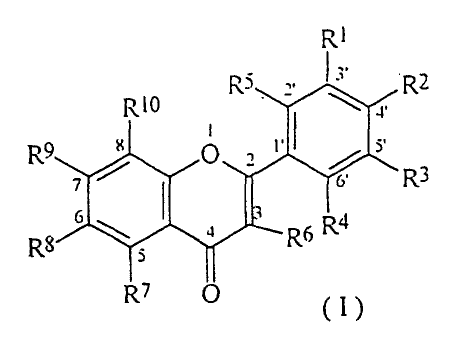 wherein
R¹, R², R³, R⁴ and R⁵ are independently selected from Cl, Br, F and H;
R⁶ is H;
R⁷, R⁹ and R¹⁰ are independently selected from H, -R, NO₂, Br, Cl, F, -OR, -NH₂, -NHR, -NR₂, -COOR, -COOH, -CN or a sugar group;
R⁸ is selected from H, NO₂, Br, Cl, and F;
R is C₁₋C₆ alkyl or alkenyl;
with the proviso that when R¹ is H, R⁸ is NO₂, and when R¹ is selected from Cl, Br, and F, R⁸ is selected from H, NO₂, Br, Cl and F
or a bi-flavonoid which is a dimer of a compound of general Formula (I) and wherein R¹ to R¹⁰ and R have the meanings given for general formula (I).

Preferred compounds of Formula (I) include compounds wherein
R¹ is selected from H, Cl and Br;
R² to R⁷ inclusive, R⁹ and R¹⁰ are all H;
R⁸ is selected from H, NO₂, Br, Cl and F; with the proviso that when R¹ is H, R⁸ is NO₂ and when R¹ is selected from Cl, Br, and F, R⁸ is selected from H, NO₂, Br, Cl and F

More preferred compounds of Formula (I) are those wherein
R¹ is selected from H and Br;
R² to R⁷ inclusive, R⁹ and R¹⁰ are all H; and
R⁸ is selected from H, NO₂ and Br; with the proviso that when R¹ is H, R⁸ is NO₂

Most preferred compounds of Formula (I) are those wherein
R¹ is Br;
R² to R⁷ inclusive, R⁹ and R¹⁰ are all H; and
R⁸ is selected from Br and NO₂.

Preferred compounds of the invention include:
6, nitroflavone 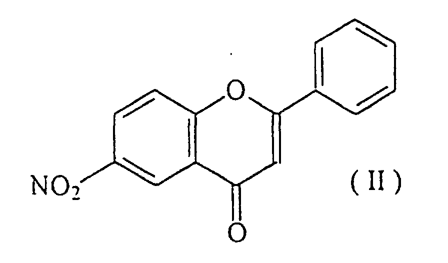
3' chloroflavone 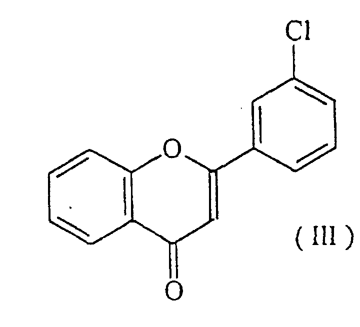
3'bromoflavone 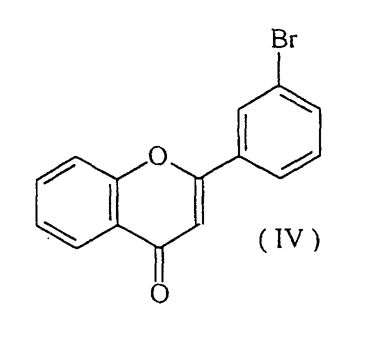
6,3'-dibromoflavone 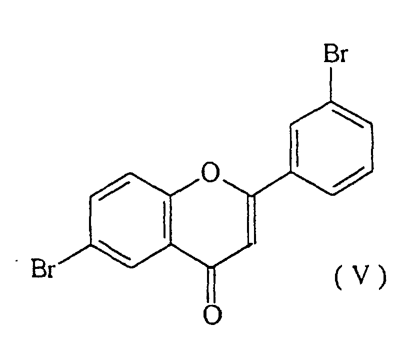
6, nitro, 3' bromoflavone

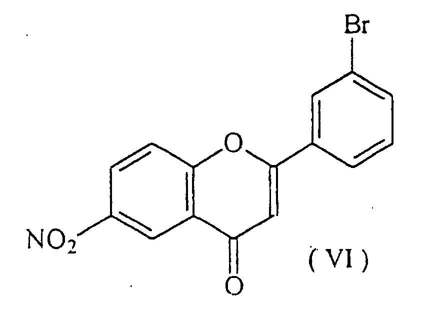

Most preferred compounds of the invention are (V) and (VI).

In a further aspect of the invention there is provided use of compounds of Formula (I) in the preparation of a medicament for the treatment of anxiety in a patient. In a preferment there is provided use of a compound according to any one or more of formulae (II) to (VII) in the preparation of a medicament for the treatment of anxiety in a patient. More preferably there is provided use of a compound or a cocktail of compounds selected from compounds (V) and (VI) in the preparation of a medicament for the treatment of anxiety in a patient.
Compounds of the invention can be prepared according to the general outline provided in scheme 1 below:

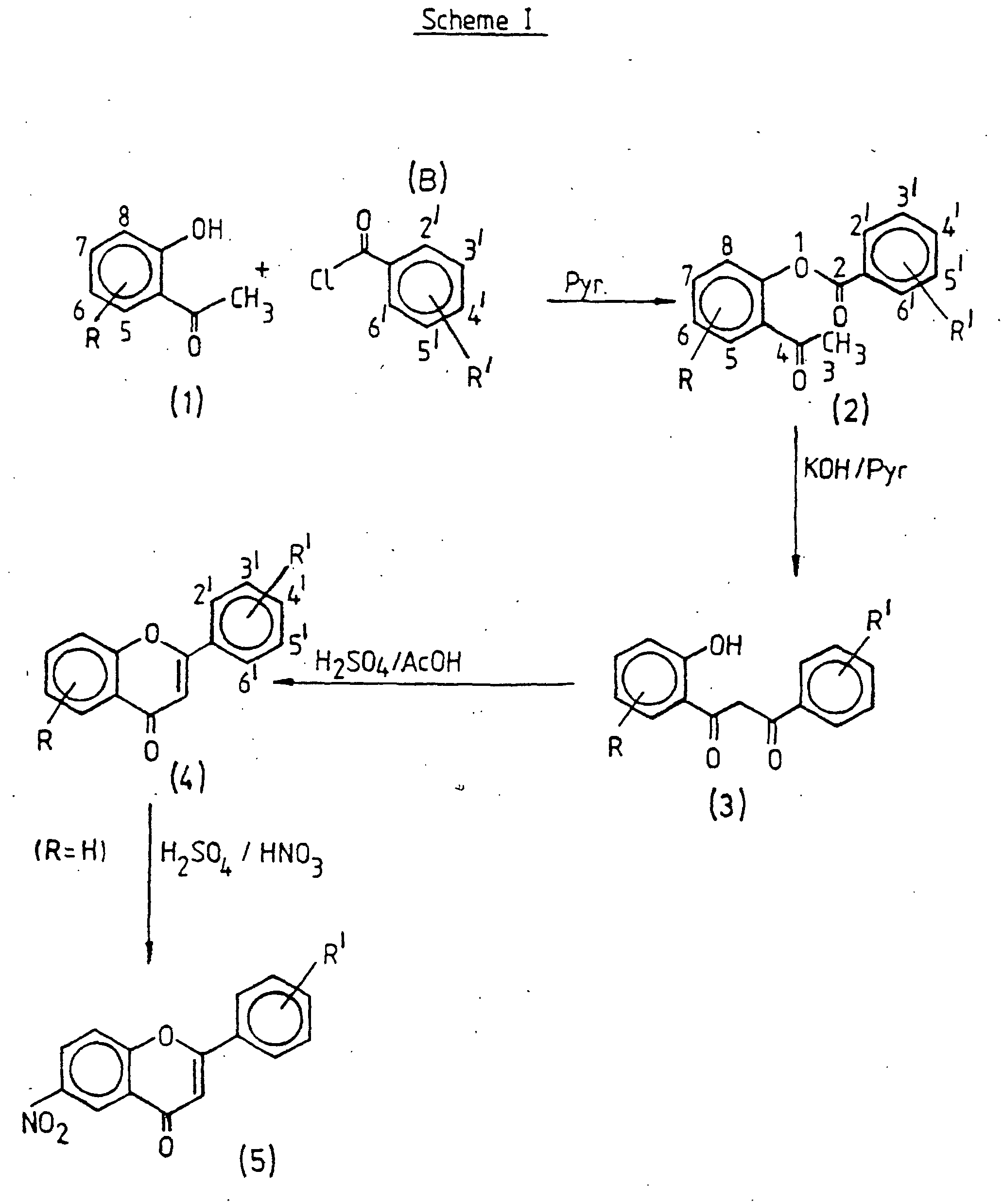

The Baker-Venkataraman transformation as described by, for example, Baker, W. Molecular rearrangement of some o-acyloxyacetophenones and the mechanism of production of 3-acylchromones. J.Chem. Soc. (1933), 1381-1388; Mahal, H.S, Venkataraman, K. Synthetical experiments in the chromone group Part XIV. The action of sodamine on 1-acyloxy-2-acetonaphthones. J. Chem. Soc. (1934), 1767-1769; Wheeler, T.S. Flavone Organic Syntheses; Wiley New York, 1963, Colleer Vol IV, pp 478-481, can be used as a method of synthesising flavone molecules of the invention.

Generally, an appropriately substituted hydroxyacetophenone (1) comprising substituents R as defined for R⁷, R⁸, R⁹ and R¹⁰ of Formula (I) can be converted into a benzoyl ester of Formula (2) by reaction with an appropriately substituted benzoyl halide (B) comprising substituents R as defined for R¹, R², R³, R⁴ and R⁵ of Formula (I). Suitable benzoyl halides include benzoyl chloride, benzoyl bromide or benzoyl fluoride having R groups as defined for R¹, R², R³, R⁴ and R⁵ above. The benzoyl ester (2) may then be treated with base, such as KOH/pyridine, forming a diketone of Formula (3) comprising R and R substituents of choice as defined above for R¹ to R⁵ inclusive and R⁷ to R¹⁰ inclusive. The diketone may then be treated with acid leading to the generation of flavone of interest (4) according to the teaching of Ares J.J. et al., A.J. Med Chem. (1995) 38 pp 4937-4943.

Where R=H, nitro-substituted flavones (5) can be obtained by treating a flavone of interest (4) with HNO₃ in H₂SO₄ using the nitration method as outlined by Cushman M. et al A.J. Med. Chem. (1994) 37 pp3353-3362.

The invention will now be illustrated by reference to the following figures and examples.
- Figure 1:: Displacement curve of [³H] FNZ (0.5nM) binding by 6-nitro-3'-bromoflavone to washed crude synaptosomal membranes from rat cerebral cortex.
- Figure 2:: Displacement curve of [³H] FNZ (0.5nM) binding by 6,3'-digromoflavone to washed crude synaptosomal membranes from rat cerebral cortex.
- Figure 3:: Displacement curve of [³H] FNZ (0.5nM) binding by 3' bromoflavone to washed crude synaptosomal membranes from rat cerebral cortex.
- Figure 4:: Displacement curve of [³H] FNZ (0.5nM) binding by 6-nitroflavone to washed crude synaptosomal membranes from rat cerebral cortex.
- Figure 5:: Displacement curve of [³H] FNZ (0.5nM) binding by 3' chloroflavone to be washed crude synaptosomal membranes from rat cerebral cortex.

### Example 1:A1:- 6' nitroflavone

Reference is made to scheme III: 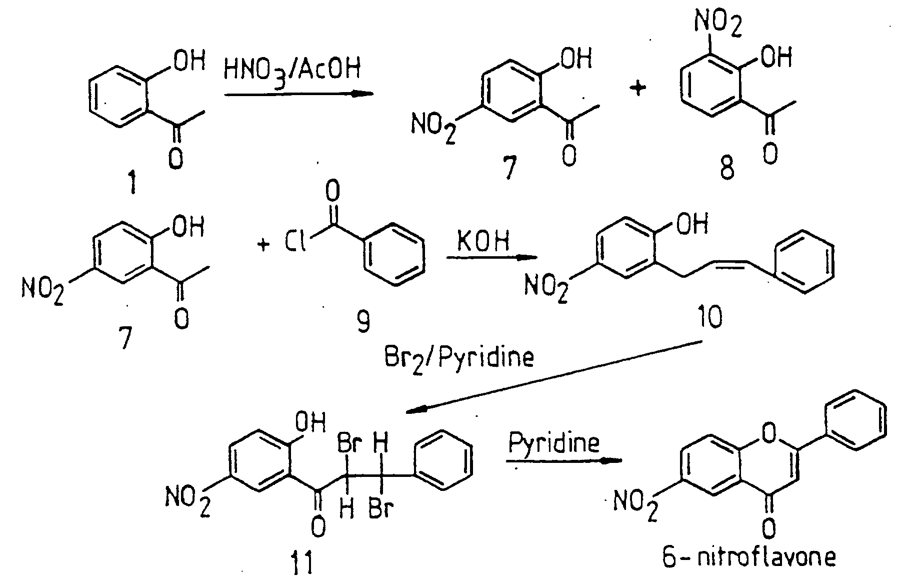 Nitric acid (fuming, 4mL) was added to a solution of 2-hydroxyacetophenone (1) (60mmol) in glacial acetic acid (70mL) and the mixture was stirred in a round-bottomed flask equipped with a drying tube (CaCl₂) at room temperature for 40 min and then at 45-50°C for 16h. Acetic acid was removed at reduced pressure, and water (100mL) was added. The yellow cloudy solution was neutralized by Na₂CO₃ to PH 6, and then the yellow precipitate was collected and carefully recrystallised from EtOH in several crops to give 7.

A solution of 5 g of compound 7 and 6 g of compound 9 in 50 mL of EtOH was treated with 50 g of KOH in 50 mL of water, and left a day at room temperature. Then it was acidified with HCland the intermediate 10 was used in the next step.

A 10% solution of bromine in 12mL of AcOH was added to a hot solution of 1 g of compound 10 in 10mL of AcOH, the mixture was left overnight at room temperature and the product crystallized from AcOH rendering compound 11. A solution of 1 g of 11 in pyridine (10mL) was stirred for 30 min at room temperature. Then it was poured into a 3% aqueous HCl/ice solution with vigorous stirring.

The resulting precipitate was filtered and washed with water. The crude material was recrystallized from ethanol yielding 6-nitroflavone.
- 6-nitroflavone:: ¹H-NMR (200 Mhz, CDCI₃): δ 9,10 (d, J=2.1) Hz, h-5), 8.53 (dd, J-2.0 Hz, 8.1 Hz, H-7), 7,96-7.91 (m, H-2, H-6'), 7.73 (d, J=8.1 Hz, H-8), 7.62-7.53 (m, H-3', H-4' H-5'), 6.67 (s, H-3).

### Example 2:A2:- 3' chloroflavone

Reference is made to scheme II:

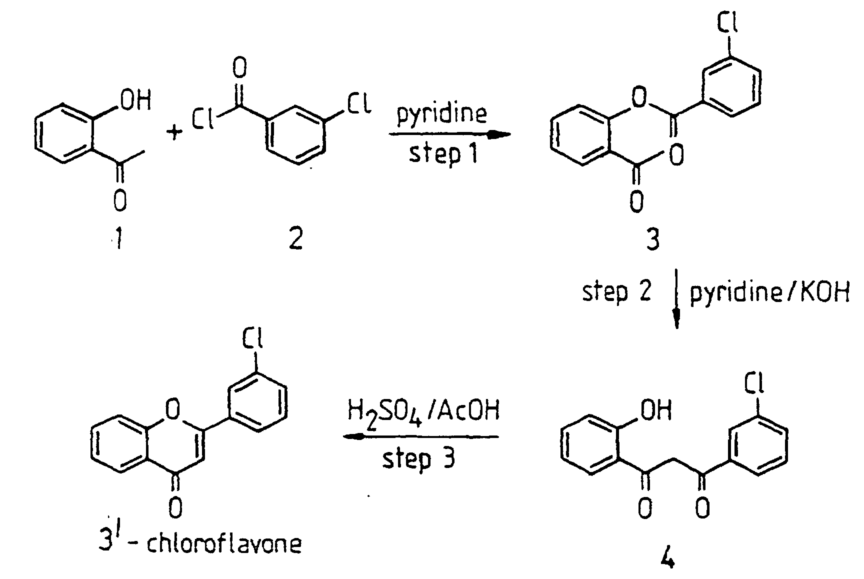

### Step 1

To a solution of the acid chloride 2 (Aldrich) (15mmol) in pyridine (10ml), at 0°C, solid 2-hydroxyacetophenone 1 (9mmol) (Aldrich) was added with stirring. The reaction mixture was stirred for 15min at 0°C followed by 30min at room temperature. Then it was poured into a 3% aqueous HCl/ice solution with vigorous stirring. The resulting precipitate was filtered and washed with water. The crude material was recrystallized from methanol yielding compound 3.

### Step 2

To a solution of compound 3 (10mmol) in pyridine (10ml), at 50°C, pulverised potassium hydroxide (15mmol) was added. The mixture was stirred for 15min and, after cooling, a 10% aqueous acetic acid solution was added. The resulting precipitate was filtered. The crude material, containing diketone 4, was used in the next step without purification.

### Step 3

A mixture of the crude material from step 2 (equivalent to 10mmol of compound 4), with concentrated sulfuric acid (0.5 ml), and glacial acetic acid (13ml) was heated at reflux for 1 h and cooled to room temperature. Then, the mixture was poured onto crushed ice (75g), and the resulting precipitate was filtered. Recrystallization from acetone afforded product 3'-chloroflavone.
- 3'-chloroflavone:: ¹H-NMR (200Mhz, CDCI₃): δ 8.24 (dd, J=2.0. Hz,8.2Hz, 11-5), 7.93 (t, J=2.2 Hz, H-2'), 7.81-7.68(m, H-4', H-6'), 7.60-7.40 (m, H-6, H-7, H-8, H-5'), 6.81 (s, H-3).

### Example 3:A3:- 3'-bromoflavone

Reference is made to scheme II (example 2)
Steps 1,2 and 3, same conditions, (Scheme II) but using compound 5 (Aldrich) and 2-hydroxyacetophenone 1 as starting materials.
- Compound 5:: 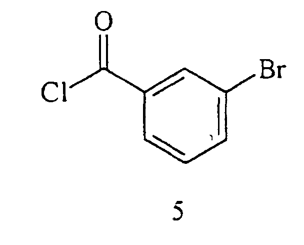
- 3'bromoflavone:: ¹H-NMR (200 Mhz, CDCI₃): δ 8.24 (dd, J=2.1, 8.5 Hz, H-5), 8.10 (t, J=2.0 Hz, H-2'), 7.84 (dq, J=2.0, 8.4 Hz, H-4'), 7.66 (m, H-6', H-6, H-7), 7.42 (m, H-5',H-8), 6.81(s,H-3).

### Example 4:A4:- 6,3'-dibromoflavone

Steps 1,2 and 3, same conditions, (scheme II example 2) but using compounds 5 and 6 (Aldrich) as starting materials. 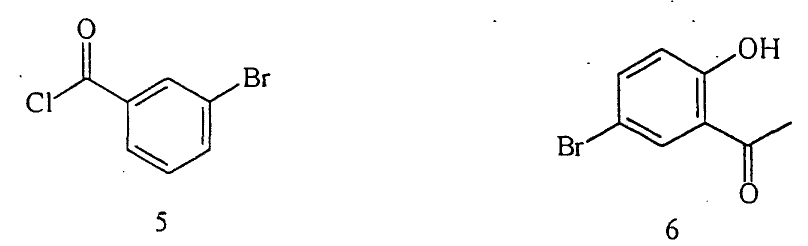
- 6,3'- dibromoflavone:: ¹H-NMR (200 Mhz, CDCl₃): δ 8.34 (s, H-2'), 8.13 (m, H-4' H-5), 8.02 (dd J=2.0, 8.2 Hz, H-7), 7.86 (d, J=8.2 Hz, H-8), 7.80 (m, H-6'), 7.55 (t, J=8.0 Hz, H-5'), 7.20 (s,H-3).

### Example 5:A5:- 6, nitro-3'-bromoflavone

### Reference is made to scheme III( example 1)

Same procedure as in Scheme III but using compounds 5 & 7 as starting materials.
- 6-nitro-3'-bromoflavone:: ¹ H-NMR (200 Mhz, CDCI₃): δ 9.11 (d, J=2.4 Hz, H=5). 8.56 (dd, J=2.4, 8.5 Hz, H-7), 8.10 (t, J=2.1 Hz, H-2'), 7.85 (m, H-4'), 7.74 (d, J=8.2 Hz, H-5'), 7.73 (m, H-6'), 7.44 (t,J=8.2 Hz, H-5'), 6.87 (s,H-3).

### Example 6: Binding Affinity of flavone derivatives for Benzodiazepine Receptors (BDZ-Rs)

### Animals

Adult male Wistar rats weighing 250g were used for biochemical experiments.

Animals were housed in a controlled environment, with free access to food and water and maintained on a 12h/12h day/night cycle.

### Membrane Preparation

Membrane preparations were carried out according to Medina et al. (1990). Briefly, brains were rapidly dissected out on ice and the different structures were homogenized in 10 volumes of 0.32 M sucrose and centrifuged at 900 x g for 10 min. The resulting supernatant was centrifuged at 100,000 x g for 30 min and the pellet washed twice in 25mM Tris HCl buffer pH 7.4 at 100,000 x g for 30 min,and stored at-20°C until used.

Binding affinities to BDZ-Rs were determined by the ability of the compounds to inhibit Hoffman-La Roche ³H-flunitrazepam (³H-FNZ) binding to extensively washed rat cerebal cortical membranes following the method as described by Levi de Stein, M. et al., Mol. Brain Res (1989),5, pp 9-15.

In brief, for each assay, triplicate samples of the membranes, containing 0.2 to 0.4 mg protein were suspended in a final volume of 1 ml of 0.25 mM Tris-HCl buffer, pH 7.3.

The incubation was carried out at 4°C for 60 min with 0.5 nM ³H-FNZ. To study the binding saturation, a range of 0.3 to 10 nM ³H-FNZ was used. Non-specific binding was determined in parallel incubations in the presence of 3µM FNZ, and represented 5-15% of total. The assays were terminated by filtration under vacuum through Whatman GF/A glass-fiber filters, and three washes with 3mL each of incubation medium. Filters were dried and counted after the addition of 5 mL of 2,5-diphenyloxazole/xylene as scintillation fluid.

Results are shown in Table 1 below.

**Table 1:**

| **Binding affinity of flavone derivatives, A1,A2,A3,A4,and A5 for Benzodiazepine receptors (BDZ-Rs) of Rat Cerebral Cortex membranes.** | | | | | | | |
|---|---|---|---|---|---|---|---|
| Compound | R⁶ | R⁸ | R⁵ | R¹ | R² | R³ | Ki^{b} (µM) |
| Flavone 6 | H | H | H | H | H | H | 1.00 |
| A1 | H | NO₂ | H | H | H | H | 0.275 |
| A2 | H | H | H | Cl | H | H | 0.614 |
| A3 | H | H | H | Br | H | H | 0.413 |
| A4 | H | Br | H | Br | H | H | 0.019 |
| A5 | H | NO, | H | Br | H | H | 0.025 |
| Ki^{b} ± SEM values are means of 3-5 independent determinations. For values ranging from 0.001 to 4.5, the SEM varied between 6 to 13% of the values listed. The low affinity values are the result of duplicate measurements. | | | | | | | |

### Biological Data

Table 2 shows the mean ± SEM of Kᵢ values of different compounds, obtained from displacement curves of [³H] flunitrazepam binding to rat synaptosomal membranes. The competition curves were analyzed using Graph-Pad Prism software.

**Table 2**

| Compound | Kᵢ (nM) | n |
|---|---|---|
| 6 nitro-3'-bromoflavone | 25.17±2.46 | 7 |
| 6,3'-dibromoflavone | 18.63±2.18 | 3 |
| 3'-bromoflavone | 413.6±46 | 3 |
| 6-nitroflavone | 275±41.1 | 3 |
| 3'-chloroflavone | 614±32.7 | 3 |
| n represents the number of experiments performed. | | |

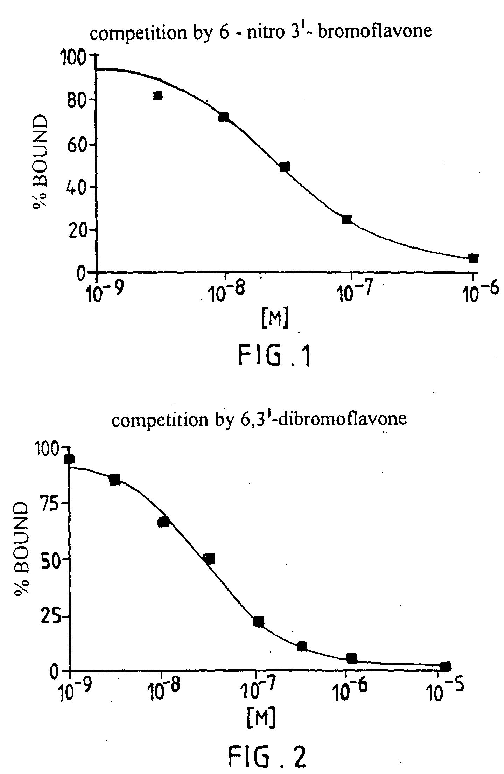

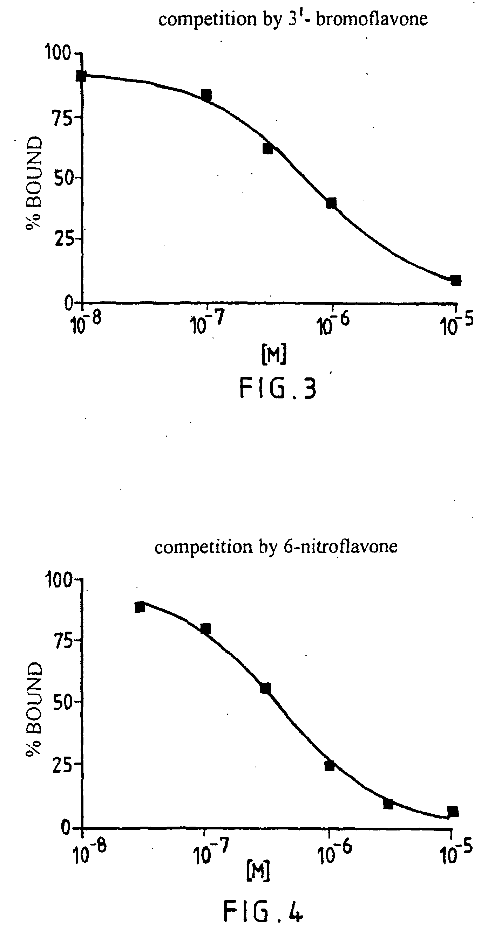

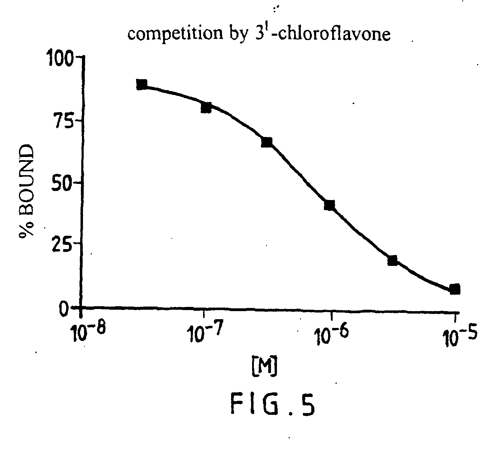

Figures 1-5 show representative displacement curves of [³H] flunitrazepam (0.5 nM) binding by different flavones to washed crude synaptosomal membranes from rat cerebral cortex. The Kᵢ's obtained in each case were:

| Compound | Kᵢ(nM) |
|---|---|
| 6-nitro-3'-bromoflavone | 21.8 |
| 6,3'-dibromoflavone | 22.7 |
| 3'-bromoflavone | 427 |
| 6-nitroflavone | 315 |
| 3'-chloroflavone | 665 |

### Results and Discussion

The following structure activity relationships were obtained by analysis of the information collected in Table 1 and are expressed as flavone substitutions that produce the following changes in the binding affinity of the mother compound (flavone 6):
A) Great increases (40 to 1000 times):
   - a bromine atom or a nitro group on carbon 6 plus a halogen group on carbon 3' (A4,A5). The effectiveness of the different substituents on carbon 6 is as follows; Br>NO₂.
B) Moderate increases (3.6 to 13 times):
   - a nitro group on carbon 6 (A1). The effectiveness of this substituent is as indicated previously.
   - a bromine or a chlorine atom on carbon 3'(A2,A3).

## Claims

1. Use of a flavonoid compound of general Formula (I) or a bi-flavonoid dimer thereof: 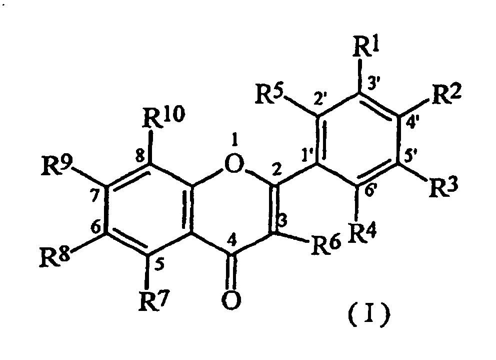 for the preparation of a medicament for the treatment of anxiety in a patient,
wherein
R¹, R², R³, R⁴ and R⁵ are independently selected from Cl, Br, F and H;
R⁶ is H;
R⁷, R⁹ and R¹⁰ are independently selected from H, -OH, -R, -NO₂, Br, Cl, F, -OR, -NH₂, -NHR, -NR₂, -COOR, -COOH, -CN or a sugar group;
R⁸ is selected from H, NO₂, Br, Cl, and F;
R is C₁₋C₆ alkyl or alkenyl;
with the provision that when R¹ is H, R⁸ is NO₂, when R¹ is selected from Cl, Br and F, R⁸ is selected from H, NO_{2,} Br, Cl and F, and at least one of R¹ to R⁵ or R⁶ to R¹⁰ is selected from the group consisting of F, Cl and Br;

2. Use of a compound according to claim 1 wherein
R¹ is selected from H, Cl and Br;
R² to R⁷ inclusive, R⁹ and R¹⁰ are all H;
R⁸ is selected from H, NO₂, Br, Cl and F.

3. Use of a compound according to claim 1 or claim 2 wherein
R¹ is selected from H and Br;
R² to R⁷ inclusive, R⁹ and R¹⁰ are all H; and
R⁸ is selected from Br, NO₂, and H.

4. Use of a compound according to any preceding claim wherein
R¹ is Br;
R² to R⁷ inclusive, R⁹ and R¹⁰ are all H; and
R⁸ is selected from Br and NO₂.

5. Use of a compound according to any one of claims 1 to 3 wherein the compound is:

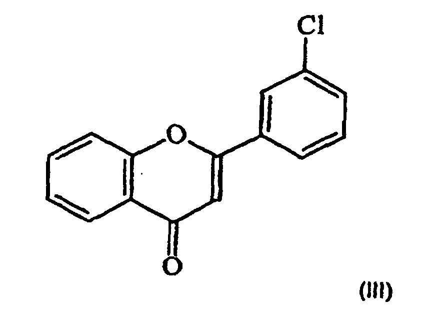

6. Use of a compound according to any one of claims 1 to 3 wherein the compound is:

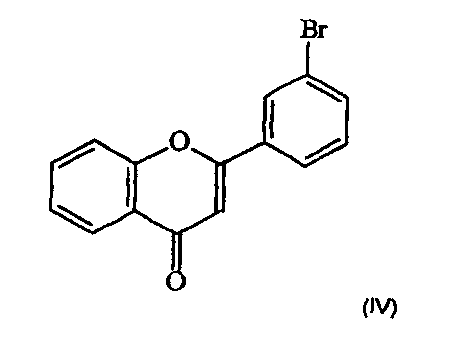

7. Use of a compound according to any one of claims 1 to 4 wherein the compound is:

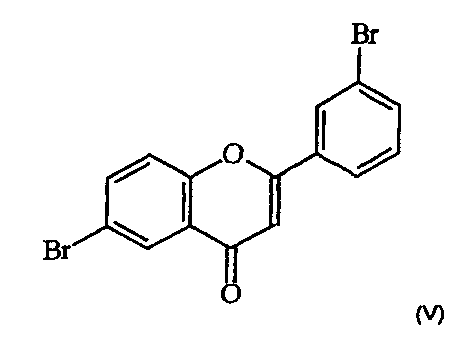

8. Use of a compound according to any one of claims 1 to 4 wherein the compound is:

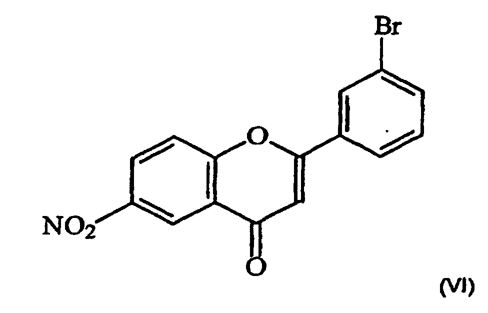

9. Use of a compound according to claim 1 wherein the bi-flavonoid dimer has the general Formula (VII): 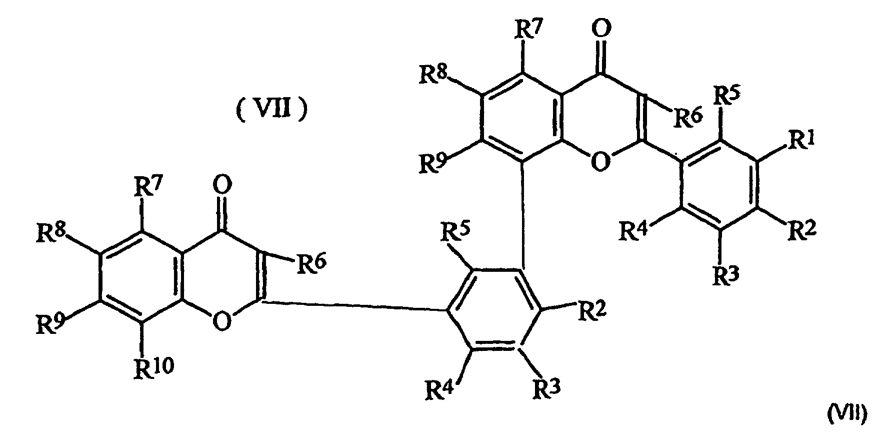 wherein R¹ to R¹⁰ have the same definitions given in claim 1.

10. Use of a compound according to claim 9 wherein
R¹ is selected from H, Cl and Br;
R² to R⁷ inclusive, R⁹ and R¹⁰ are all H;
R⁸ is selected from H, NO₂, Br, Cl and F.

11. Use of a compound according to claim 9 or claim 10 wherein
R¹ is selected from H and Br;
R² to R⁷ inclusive, R⁹ and R¹⁰ are all H; and
R⁸ is selected from Br, NO₂, and H.

12. Use of a compound according to any one of claims 9 to 11 wherein
R¹ is Br;
R² to R⁷ inclusive, R⁹ and R¹⁰ are all H; and
R⁸ is selected from Br and NO₂.

13. Use of a compound according to any of the preceding claims wherein the treatment reduces anxiety without exerting a substantially sedative effect

14. A pharmaceutical formulation which comprises a flavonoid of Formula (I) or a bi-flavonoid dimer thereof in admixture with a pharmaceutically acceptable carrier 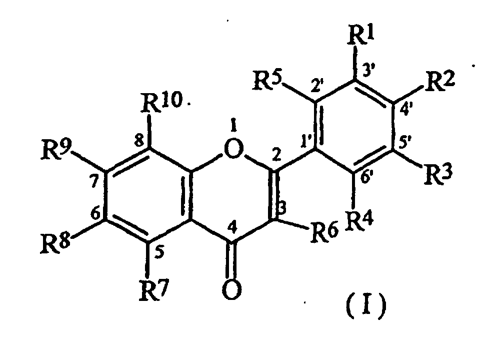 wherein
R¹, R², R³, R⁴ and R⁵ are independently selected from Cl, Br, F and H;
R⁶ is H;
R⁷, R⁹ and R¹⁰ are independently selected from H, -OH, -R, - NO₂, Br, Cl, F, -OR, -NN₂, -NHR, -NR₂, -COOR, -COOH, -CN or a sugar group;
R⁸ is selected from H, NO₂, Br, Cl, and F;
R is C₁₋C₆ alkyl or alkenyl;
with the proviso that when R¹ is H, R⁸ is NO₂, when
R¹ is selected from Cl, Br and F, R⁸ is selected from H, NO₂, Br, Cl and F, and at least one of R¹ to R⁵ or R⁶ to R¹⁰ is selected from the group consisting of F, Cl and Br.

15. A formulation according to claim 14 wherein
R¹ is selected from H, Cl and Br;
R² to R⁷ inclusive, R⁹ and R¹⁰ are all H;
R⁸ is selected from H, NO₂, Br, Cl and F.

16. A formulation according to claim 14 or claim 15 wherein
R¹ is selected from H and Br;
R² to R⁷ inclusive, R⁹ and R¹⁰ are all H; and
R⁸ is selected from Br, NO₂, and H.

17. A formulation according to any one of claims 14 to 16 wherein
R¹ is Br;
R² to R⁷ inclusive, R⁹ and R¹⁰ are all H; and
R⁸ is selected from Br and NO₂.

18. A formulation according to claim 14 wherein the compound of general Formula (I) is:

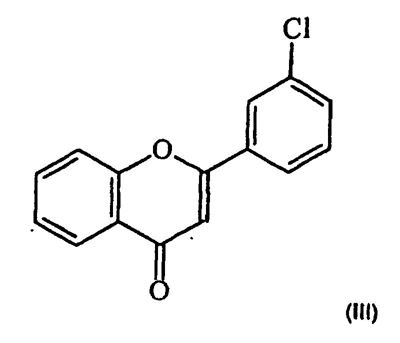

19. A formulation according to claim 14 wherein the compound of general Formula (I) is:

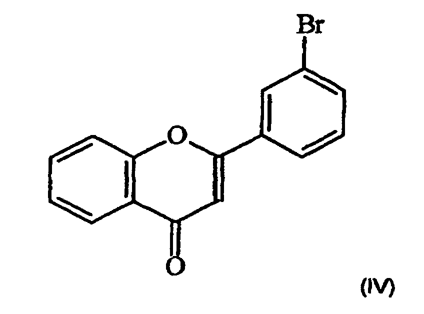

20. A formulation according to claim 14 wherein the compound of general Formula (I) is:

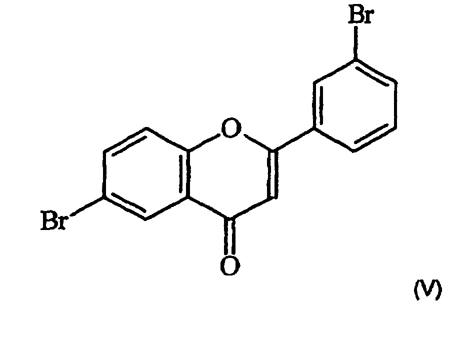

21. A formulation according to claim 14 wherein the compound of general Formula (I) is:

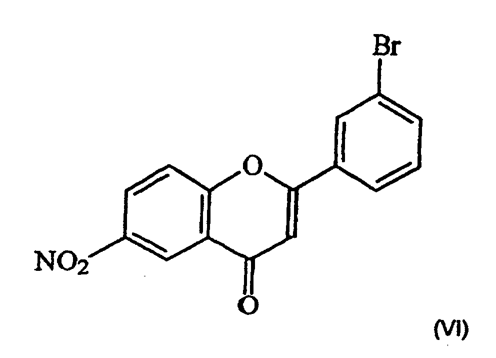

22. A formulation according to claim 14 wherein the bi-flavonoid dimer is of general Formula (VII): 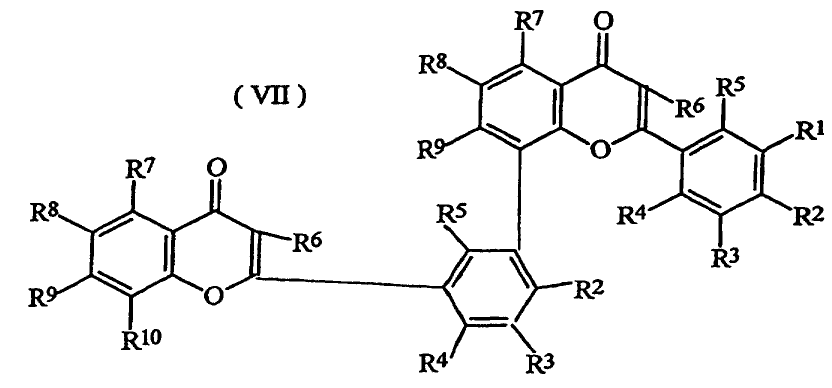 wherein R¹ to R¹⁰ and R have the meaning given in claim 14.

23. A formulation according to claim 22 wherein
R¹ is selected from H, Cl and Br;
R⁸ is selected from Cl, Br, F, H and NO₂; and
R², R³, R⁴, R⁵, R⁶ and R⁷ are as defined for formula (I).

24. A formulation according to claim 22 wherein
R¹ is selected from Br, Cl or F;
R⁸ is selected from NO₂, Cl and Br; and
all other R groups are H.

25. A formulation according to claim 24 wherein the bi-flavonoid dimer is a dimer of monomers selected from compounds III, IV, V or VI.

26. A flavonoid compound of general Formula (I): 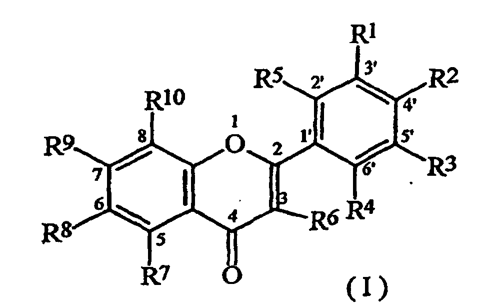 wherein
R¹, R², R³, R⁴ and R⁵ are independently selected from Cl, Br, F and H;
R⁶ is H;
R⁷, R⁹ and R¹⁰ are independently selected from H, -R, NO₂, Br, Cl, F, -OR, -NH₂, -NHR, -NR₂, -COOR, -COOH, -CN or a sugar group;
R⁸ is selected from H, NO₂, Br, Cl, and F;
R is C₁₋C₆ alkyl or alkenyl;
with the proviso that when R¹ is H, R⁸ is NO₂, when R¹ is selected from Cl, Br, and F, R⁸ is selected from H, NO₂, Br, Cl and F, and at least one of R¹ to R⁵ or R⁸ to R¹⁰ is selected from the group consisting of F, Cl and Br;
or a bi-flavonoid which is a dimer of a compound of general Formula (I) and wherein R¹ to R¹⁰ and R have the meanings given for general Formula (I).

27. A flavonoid compound according to claim 26 wherein
R¹ is selected from H, Cl and Br;
R² to R⁷ inclusive, R⁹ and R¹⁰ are all H;
R⁸ is selected from H, NO₂ Br, Cl and F.

28. A flavonoid compound according to claim 26 or claim 27 wherein
R¹ is selected from H and Br;
R² to R⁷ inclusive, R⁹ and R¹⁰ are all H; and
R⁸ is selected from H, NO₂ and Br.

29. A flavonoid compound according to any one of claims 26 to 28 wherein
R¹ is Br;
R² to R⁷ inclusive, R⁹ and R¹⁰ are all H; and
R⁸ is selected from Br and NO₂.

30. A flavonoid compound according to claim 26 which is selected from compounds III, IV, V and VI.

31. A flavonoid compound according to claim 26 which is:

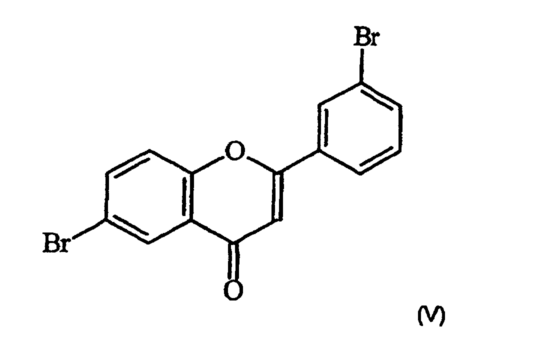

32. A flavonoid compound according to claim 26 which is:

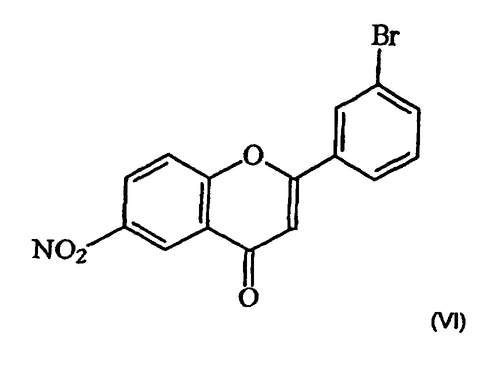

33. Use of a mixture of compounds selected from the compounds III, IV, V and VI and/or bi-flavonoid dimers thereof for the preparation of a medicament for the treatment of anxiety in a patient.

34. Use of a mixture of compounds according to claim 33, wherein said compounds are selected from compounds V and VI and/or bi-flavonoid dimers thereof.

## Patentansprüche

1. Verwendung einer Flavonoid-Verbindung der allgemeinen Formel (I) oder eines Bi-Flavonoid-Dimers davon: 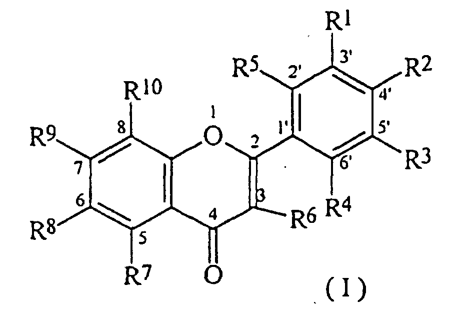 für die Herstellung eines Medikaments für die Behandlung von Angstzuständen in einem Patienten, in welcher Formel sind:
R¹, R², R³, R⁴ und R⁵ unabhängig ausgewählt aus Cl, Br, F und H;
R⁶ H;
R⁷, R⁹ und R¹⁰ unabhängig ausgewählt aus H, -OH, -R, -NO₂, Br, Cl, F, -OR, -NH₂, -NHR, -NR₂, -COOR, -COOH, -CN oder einer Zuckergruppe;
R⁸ ausgewählt aus H, NO₂, Br, Cl und F;
R C₁-C₆-Alkyl oder -Alkenyl;
unter der Voraussetzung, dass, wenn R¹ H ist, R⁸ NO₂ ist und wenn R¹ ausgewählt ist aus Cl, Br und F, ist R⁸ ausgewählt aus H, NO₂, Br, Cl und F, wobei mindestens ein Vertreter von R¹ bis R⁵ oder R⁶ bis R¹⁰ ausgewählt ist aus der Gruppe, bestehend aus F, Cl und Br.

2. Verwendung einer Verbindung nach Anspruch 1, worin sind:
R¹ ausgewählt aus H, Cl und Br;
R² bis R⁷ einschließlich, R⁹ und R¹⁰ insgesamt alle H;
R⁸ ausgewählt aus H, NO₂, Br, Cl und F.

3. Verwendung einer Verbindung nach Anspruch 1 oder Anspruch 2, worin sind:
R¹ ausgewählt aus H und Br;
R² bis R⁷ einschließlich, R⁹ und R¹⁰ alle H; und
R⁸ ausgewählt aus Br, NO₂ und H.

4. Verwendung einer Verbindung nach einem der vorgenannten Ansprüche, worin sind:
R¹ Br;
R² bis R⁷ einschließlich, R⁹ und R¹⁰ alle H; und
R⁸ ausgewählt aus Br und NO₂.

5. Verwendung einer Verbindung nach einem der Ansprüche 1 bis 3, worin die Verbindung die Formel hat:

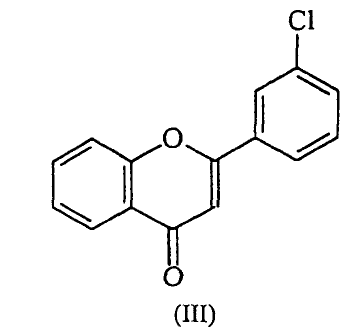

6. Verwendung einer Verbindung nach einem der Ansprüche 1 bis 3, worin die Verbindung die Formel hat:

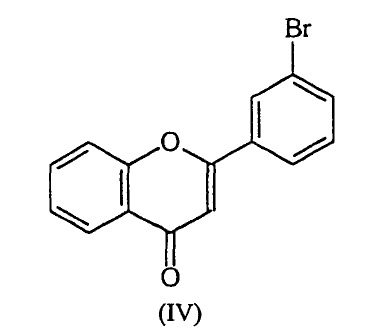

7. Verwendung einer Verbindung nach einem der Ansprüche 1 bis 4, worin die Verbindung die Formel hat:

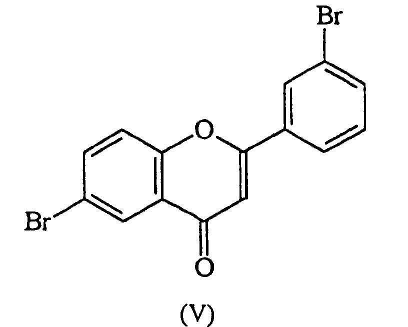

8. Verwendung einer Verbindung nach einem der Ansprüche 1 bis 4, worin die Verbindung die Formel hat:

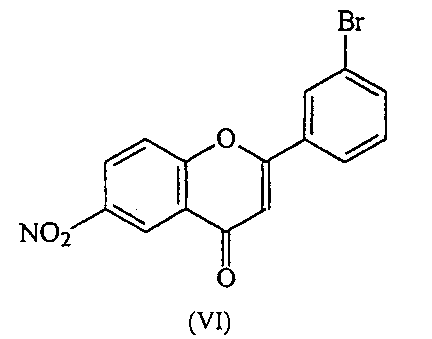

9. Verwendung einer Verbindung nach Anspruch 1, worin das Bi-Flavonoid-Dimer die allgemeine Formel (VII) hat: 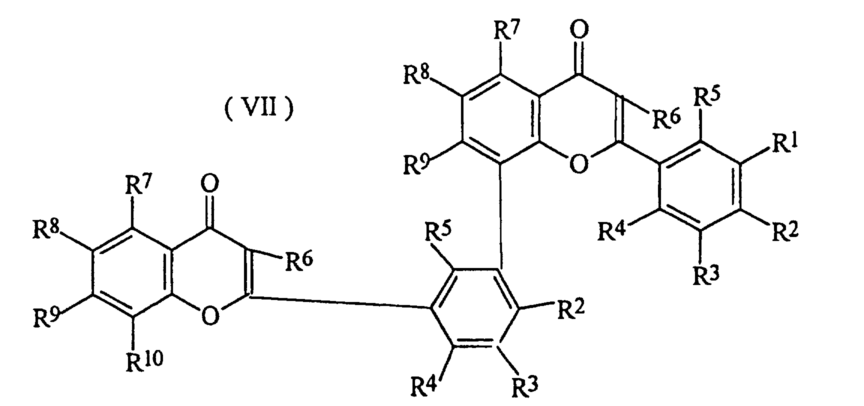 worin R¹ bis R¹⁰ die gleichen Definitionen haben, wie in Anspruch 1.

10. Verwendung einer Verbindung nach Anspruch 9, worin sind:
R¹ ausgewählt aus H, Cl und Br;
R² bis R⁷ einschließlich, R⁹ und R¹⁰ insgesamt alle H;
R⁸ ausgewählt aus H, NO₂, Br, Cl und F.

11. Verwendung einer Verbindung nach Anspruch 9 oder Anspruch 10, worin sind:
R¹ ausgewählt aus H und Br;
R² bis R⁷ einschließlich, R⁹ und R¹⁰ insgesamt alle H; und
R⁸ ausgewählt aus Br, NO₂ und H.

12. Verwendung einer Verbindung nach einem der Ansprüche 9 bis 11, worin sind:
R¹ Br;
R² bis R⁷ einschließlich, R⁹ und R¹⁰ insgesamt alle H;
R⁸ ausgewählt aus Br und NO₂.

13. Verwendung einer Verbindung nach einem der vorgenannten Ansprüche, worin die Behandlung Angstzustände ohne Ausübung einer sedativen Wirkung verringert.

14. Pharmazeutische Formulierung, welche ein Flavonoid der Formel (I) oder ein Bi-Flavonoid-Dimer davon in Zumischung mit einem pharmazeutisch zulässigen Träger aufweist: 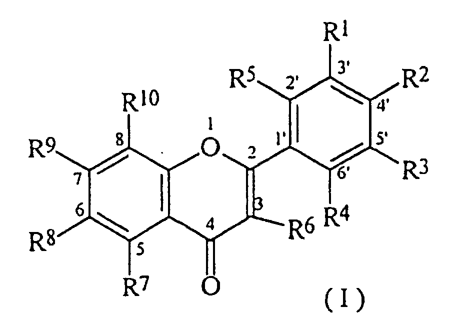 worin sind:
R¹, R², R³, R⁴ und R⁵ unabhängig ausgewählt aus Cl, Br, F und H;
R⁶ H;
R⁷, R⁹ und R¹⁰ unabhängig ausgewählt aus H, -OH, -R, -NO₂, Br, Cl, F, -OR, -NH₂, -NHR, -NR₂, -COOR, -COOH, -CN oder einer Zuckergruppe;
R⁸ ausgewählt aus H, NO₂, Br, Cl und F;
R C₁-C₆-Alkyl oder -Alkenyl;
unter der Voraussetzung, dass, wenn R¹ H ist, R⁸ NO₂ ist, und wenn R¹ ausgewählt ist aus Cl, Br und F, ist R⁸ ausgewählt aus H, NO₂, Br, Cl und F, wobei mindestens ein Vertreter von R¹ bis R⁵ oder R⁶ bis R¹⁰ ausgewählt ist aus der Gruppe, bestehend aus F, Cl und Br.

15. Formulierung nach Anspruch 14, worin sind:
R¹ ausgewählt aus H, Cl und Br;
R² bis R⁷ einschließlich, R⁹ und R¹⁰ insgesamt alle H;
R⁸ ausgewählt aus H, NO₂, Br, Cl und F.

16. Formulierung nach Anspruch 14 oder Anspruch 15, worin sind:
R¹ ausgewählt aus H und Br;
R² bis R⁷ einschließlich, R⁹ und R¹⁰ insgesamt alle H;
R⁸ ausgewählt aus Br, NO₂ und H.

17. Formulierung nach einem der Ansprüche 14 bis 16, worin sind:
R¹ Br;
R² bis R⁷ einschließlich, R⁹ und R¹⁰ insgesamt alle H;
R⁸ ausgewählt aus Br und NO₂.

18. Formulierung nach Anspruch 14, worin die Verbindung der allgemeinen Formel (I) lautet:

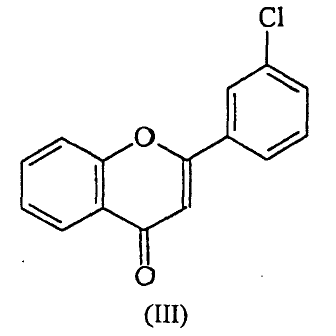

19. Formulierung nach Anspruch 14, worin die Verbindung der allgemeinen Formel (I) lautet:

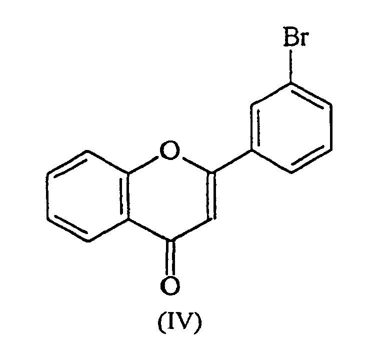

20. Formulierung nach Anspruch 14, worin die Verbindung der allgemeinen Formel (I) lautet:

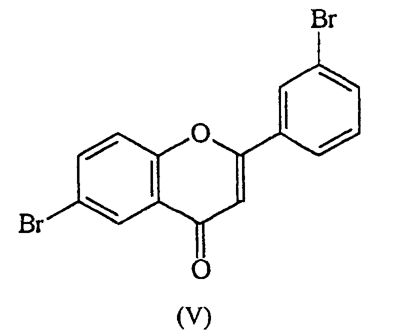

21. Formulierung nach Anspruch 14, worin die Verbindung der allgemeinen Formel (I) lautet:

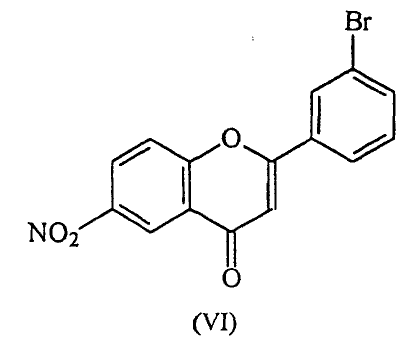

22. Formulierung nach Anspruch 14, worin das Bi-Flavonoid-Dimer die allgemeine Formel (VII) hat: 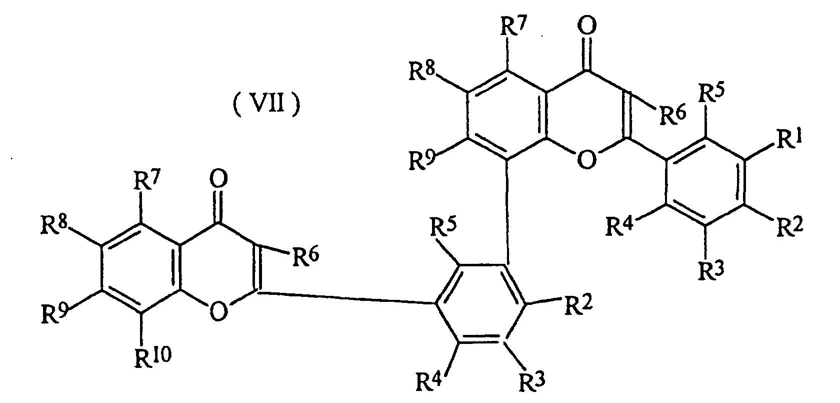 worin R¹ bis R¹⁰ und R die in Anspruch 14 festgelegte Bedeutung haben.

23. Formulierung nach Anspruch 22, worin sind:
R¹ ausgewählt aus H, Cl und Br;
R⁶ ausgewählt aus Cl, Br, F, H und NO₂; und
R², R³, R⁴, R⁵, R⁶ und R⁷ wie für Formel (I) festgelegt.

24. Formulierung nach Anspruch 22, worin sind:
R¹ ausgewählt aus Br, Cl oder F;
R⁸ ausgewählt aus NO₂, Cl und Br; und
alle anderen R-Gruppen sind H.

25. Formulierung nach Anspruch 24, worin das Bi-Flavonoid-Dimer ein Dimer von Monomeren ist, ausgewählt aus Verbindungen III, IV, V oder VI.

26. Flavonoid-Verbindung der allgemeinen Formel (I): 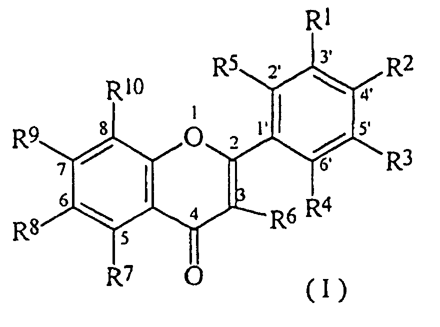 worin sind:
R¹, R², R³, R⁴ und R⁵ unabhängig ausgewählt aus Cl, Br, F und H;
R⁶ H;
R⁷, R⁹ und R¹⁰ unabhängig ausgewählt aus H, -R, NO₂, Br, Cl, F, -OR, -NH₂, -NHR, -NR₂, -COOR, -COOH, -CN oder einer Zuckergruppe;
R⁸ ausgewählt aus H, NO₂, Br, Cl und F;
R C₁-C₆-Alkyl oder -Alkenyl;
unter der Voraussetzung, dass, wenn R¹ H ist, R⁸ NO₂ ist, und wenn R¹ ausgewählt ist aus Cl, Br und F, ist R⁸ ausgewählt aus H, NO₂, Br, Cl und F, wobei mindestens ein Vertreter von R¹ bis R⁶ oder R⁶ bis R¹⁰ ausgewählt ist aus der Gruppe, bestehend aus F, Cl und Br;
oder ein Bi-Flavonoid, das ein Dimer einer Verbindung der allgemeinen Formel (I) ist und worin R¹ bis R¹⁰ und R die für die allgemeine Formel (I) festgelegten Bedeutungen haben.

27. Flavonoid-Verbindung nach Anspruch 26, worin sind:
R¹ ausgewählt aus H, Cl und Br;
R² bis R⁷ einschließlich, R⁹ und R¹⁰ insgesamt alle H;
R⁸ ausgewählt aus H, NO₂, Br, Cl und F.

28. Flavonoid-Verbindung nach Anspruch 26 oder Anspruch 27, worin sind:
R¹ ausgewählt aus H und Br;
R² bis R⁷ einschließlich, R⁹ und R¹⁰ insgesamt alle H; und
R⁸ ausgewählt aus H, NO₂ und Br.

29. Flavonoid-Verbindung nach einem der Ansprüche 26 bis 28, worin sind:
R¹ Br;
R² bis R⁷ einschließlich, R⁹ und R¹⁰ insgesamt alle H; und
R⁸ ausgewählt aus Br und NO₂.

30. Flavonoid-Verbindung nach Anspruch 26, die ausgewählt ist aus den Verbindungen III, IV, V und VI.

31. Flavonoid-Verbindung nach Anspruch 26, die lautet:

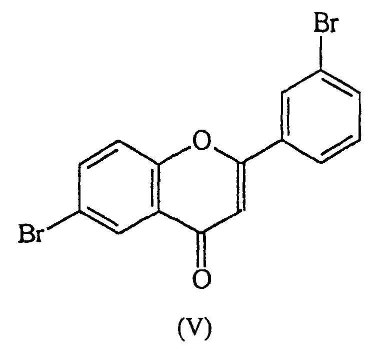

32. Flavonoid-Verbindung nach Anspruch 26, die lautet:

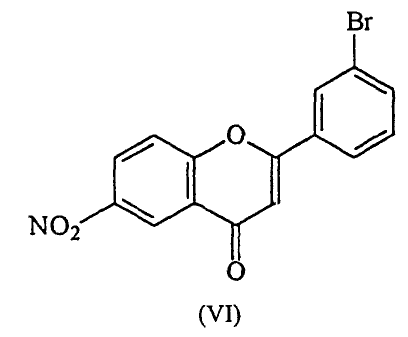

33. Verwendung einer Mischung von Verbindungen, ausgewählt aus den Verbindungen III, IV, V und VI und/oder Bi-Flavonoid-Dimeren davon für die Herstellung eines Medikaments für die Behandlung von Angstzuständen in einem Patienten.

34. Verwendung einer Mischung von Verbindungen nach Anspruch 33, worin die Verbindungen ausgewählt sind aus den Verbindungen V und VI und/oder Bi-Flavonoid-Dimeren davon.

## Revendications

1. Utilisation d'un composé de flavonoïde de la formule générale (I) ou d'un dimère de bi-flavonoïde de celui-ci: 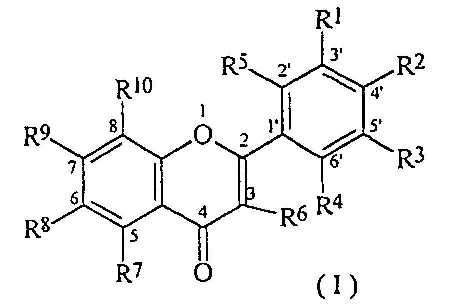 pour la préparation d'un médicament pour le traitement de l'anxiété chez un patient,
dans laquelle formule:
R¹, R², R³, R⁴ et R⁵ sont indépendamment choisis parmi Cl, Br, F et H;
R⁶ est H;
R⁷, R⁹ et R¹⁰ sont indépendamment choisis parmi H, -OH, -R, -NO₂, Br, Cl, F, -OR, -NH₂, -NHR, -NR₂, -COOR, -COOH, -CN ou un groupe de sucre;
R⁸ est choisi parmi H, NO₂, Br, Cl et F;
R est un groupe alkyle ou alcényle C₁-C₆;
sous réserve que, lorsque R¹ est H, R⁸ soit NO₂, lorsque R¹ est choisi parmi Cl, Br et F, R⁸ soit choisi parmi H, NO₂, Br, Cl et F, et au moins un de R¹ à R⁵ ou de R⁶ à R¹⁰ soit choisi dans le groupe constitué de F, Cl et Br.

2. Utilisation d'un composé suivant la revendication 1, dans lequel:
R' est choisi parmi H, Cl et Br;
R² à R⁷ inclus, R⁹ et R¹⁰ sont tous H;
R⁸ est choisi parmi H, NO₂, Br, Cl et F.

3. Utilisation d'un composé suivant la revendication 1 ou la revendication 2, dans lequel:
R¹ est choisi parmi H et Br;
R² à R⁷ inclus, R⁹ et R¹⁰ sont tous H; et
R⁸ est choisi parmi Br, NO₂ et H.

4. Utilisation d'un composé suivant l'une quelconque des revendications précédentes, dans lequel:
R¹ est Br;
R² à R⁷ inclus, R⁹ et R¹⁰ sont tous H; et
R⁸ est choisi parmi Br et NO₂.

5. Utilisation d'un composé suivant l'une quelconque des revendications 1 à 3, dans laquelle le composé est:

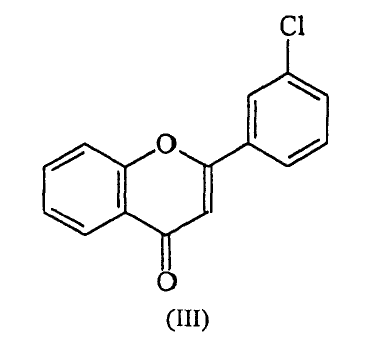

6. Utilisation d'un composé suivant l'une quelconque des revendications 1 à 3, dans laquelle le composé est:

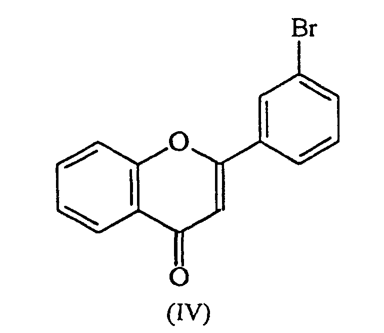

7. Utilisation d'un composé suivant l'une quelconque des revendications 1 à 4, dans laquelle le composé est:

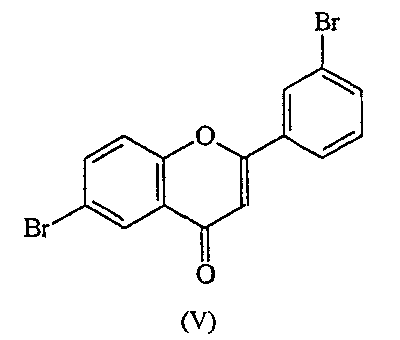

8. Utilisation d'un composé suivant l'une quelconque des revendications 1 à 4, dans laquelle le composé est:

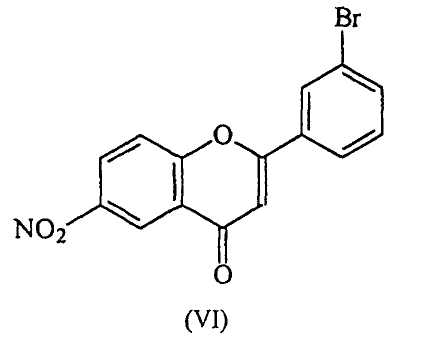

9. Utilisation d'un,composé suivant la revendication 1, dans laquelle le dimère de bi-flavonoïde présente la formule générale (VII): 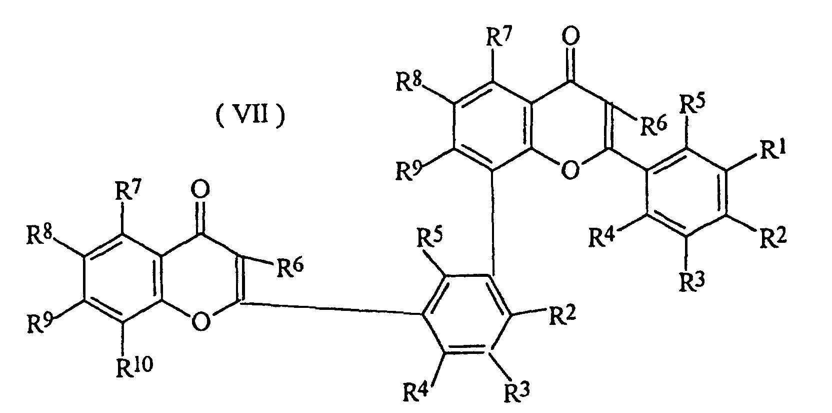 dans laquelle R¹ à R¹⁰ possèdent les mêmes définitions que celles données dans la revendication 1.

10. Utilisation d'un composé suivant la revendication 9, dans lequel:
R¹ est choisi parmi H, Cl et Br;
R² à R⁷ inclus, R⁹ et R¹⁰ sont tous H;
R⁸ est choisi parmi H, NO₂, Br, Cl et F.

11. Utilisation d'un composé suivant la revendication 9 ou la revendication 10, dans lequel:
R¹ est choisi parmi H et Br;
R² à R⁷ inclus, R⁹ et R¹⁰ sont tous H; et
R⁸ est choisi parmi Br, NO₂ et H.

12. Utilisation d'un composé suivant l'une quelconque des revendications 9 à 11, dans lequel:
R¹ est Br;
R² à R⁷ inclus, R⁹ et R¹⁰ sont tous H; et
R⁸ est choisi parmi Br et NO₂.

13. Utilisation d'un composé suivant l'une quelconque des revendications précédentes, dans laquelle le traitement réduit l'anxiété sans exercer d'effet substantiellement sédatif.

14. Formulation pharmaceutique qui comprend un flavonoïde de la formule (I) ou un dimère de bi-flavonoïde de celui-ci dans un mélange avec un excipient pharmaceutiquement acceptable: 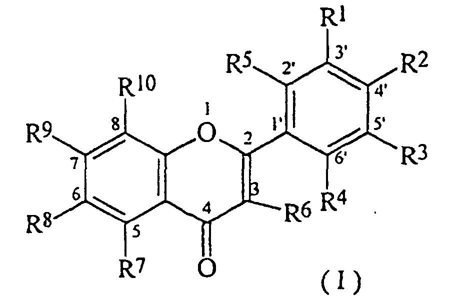 dans laquelle formule:
R¹, R², R³, R⁴ et R⁵ sont indépendamment choisis parmi Cl, Br, F et H;
R⁶ est H;
R⁷, R⁹ et R¹⁰ sont indépendamment choisis parmi H, -OH, -R, -NO₂, Br, Cl, F, -OR, -NH₂, -NHR, -NR₂, -COOR, -COOH, -CN ou un groupe de sucre;
R⁸ est choisi parmi H, NO₂, Br, Cl et F;
R est un groupe alkyle ou alcényle C₁-C₆;
sous réserve que, lorsque R¹ est H, R⁸ soit NO₂, lorsque R¹ est choisi parmi Cl, Br et F, R⁸ soit choisi parmi H, NO₂, Br, Cl et F, et au moins un de R¹ à R⁵ ou de R⁶ à R¹⁰ soit choisi dans le groupe constitué de F, Cl et Br.

15. Formulation suivant la revendication 14, dans laquelle:
R¹ est choisi parmi H, Cl et Br;
R² à R⁷ inclus, R⁹ et R¹⁰ sont tous H;
R⁸ est choisi parmi H, NO₂, Br, Cl et F.

16. Formulation suivant la revendication 14 ou la revendication 15, dans laquelle:
R¹ est choisi parmi H et Br;
R² à R⁷ inclus, R⁹ et R¹⁰ sont tous H; et
R⁸ est choisi parmi Br, NO₂ et H.

17. Formulation suivant l'une quelconque des revendications 14 à 16, dans laquelle:
R¹ est Br;
R² à R⁷ inclus, R⁹ et R¹⁰ sont tous H; et
R⁸ est choisi parmi Br et NO₂.

18. Formulation suivant la revendication 14, dans laquelle le composé de la formule générale (I) est:

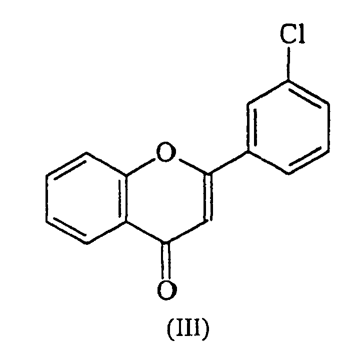

19. Formulation suivant la revendication 14, dans laquelle le composé de la formule générale (I) est:

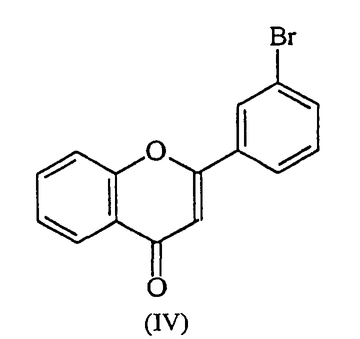

20. Formulation suivant la revendication 14, dans laquelle le composé de la formule générale (I) est:

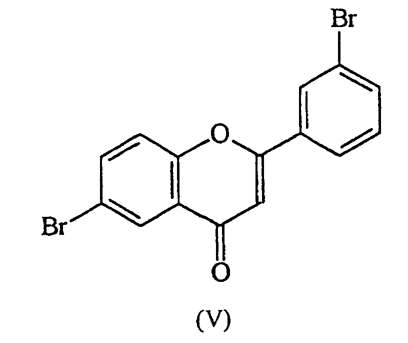

21. Formulation suivant la revendication 14, dans laquelle le composé de la formule générale (I) est:

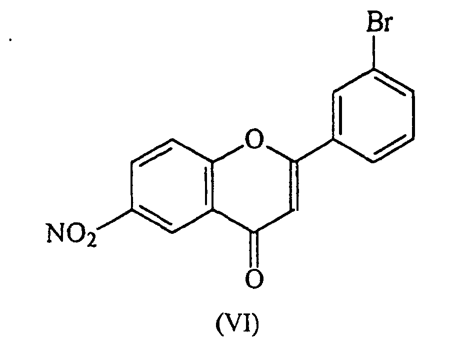

22. Formulation suivant la revendication 14, dans laquelle le dimère de bi-flavonoïde est de la formule générale (VII): 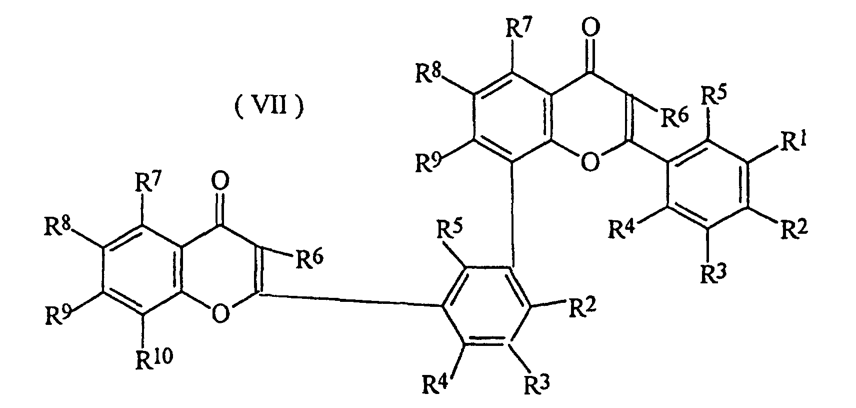 dans laquelle R¹ à R¹⁰ et R possèdent la signification donnée dans la revendication 14.

23. Formulation suivant la revendication 22, dans laquelle:
R¹ est choisi parmi H, Cl et Br;
R⁸ est choisi parmi Cl, Br, F, H et NO₂; et
R², R³, R⁴, R⁵, R⁶ et R⁷ sont tels que définis pour la formule (I).

24. Formulation suivant la revendication 22, dans laquelle:
R¹ est choisi parmi Br, Cl ou F;
R⁸ est choisi parmi NO₂, Cl et Br; et
tous les autres groupes R sont H.

25. Formulation suivant la revendication 24, dans laquelle le dimère de bi-flavonoïde est un dimère de monomères choisis parmi les composés III, IV, V ou VI.

26. Composé de flavonoïde de la formule générale (I): 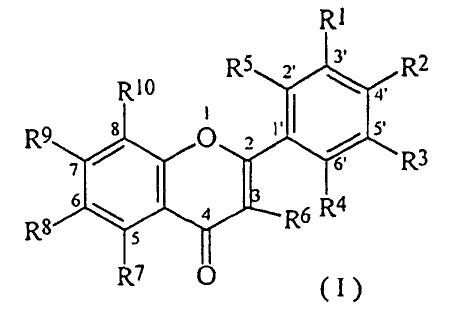 dans laquelle:
R¹, R², R³, R⁴ et R⁵ sont indépendamment choisis parmi Cl, Br, F et H;
R⁶ est H;
R⁷, R⁹ et R¹⁰ sont indépendamment choisis parmi H, -R, -NO₂, Br, Cl, F, -OR, -NH₂, -NHR, -NR₂, -COOR, -COOH, -CN ou un groupe de sucre;
R⁸ est choisi parmi H, NO₂, Br, Cl et F;
R est un groupe alkyle ou alcényle C₁-C₆;
sous réserve que, lorsque R¹ est H, R⁸ soit NO₂, lorsque R¹ est choisi parmi Cl, Br et F, R⁸ soit choisi parmi H, NO₂, Br, Cl et F, et au moins un de R¹ à R⁵ ou de R⁶ à R¹⁰ soit choisi dans le groupe constitué de F, Cl et Br,
ou bi-flavonoïde qui est un dimère d'un composé de la formule générale (I) et dans laquelle R¹ à R¹⁰ et R possèdent les significations données pour la formule générale (I).

27. Composé de flavonoïde suivant la revendication 26, dans lequel:
R¹ est choisi parmi H, Cl et Br;
R² à R⁷ inclus, R⁹ et R¹⁰ sont tous H;
R⁸ est choisi parmi H, NO₂, Br, Cl et F.

28. Composé de flavonoïde suivant la revendication 26 ou la revendication 27, dans lequel:
R¹ est choisi parmi H et Br;
R² à R⁷ inclus, R⁹ et R¹⁰ sont tous H; et
R⁸ est choisi parmi H, NO₂ et Br.

29. Composé de flavonoïde suivant l'une quelconque des revendications 26 à 28, dans lequel:
R¹ est Br;
R² à R⁷ inclus, R⁹ et R¹⁰ sont tous H; et
R⁸ est choisi parmi Br et NO₂.

30. Composé de flavonoïde suivant la revendication 26, qui est choisi parmi les composés III, IV, V et VI.

31. Composé de flavonoïde suivant la revendication 26, qui est:

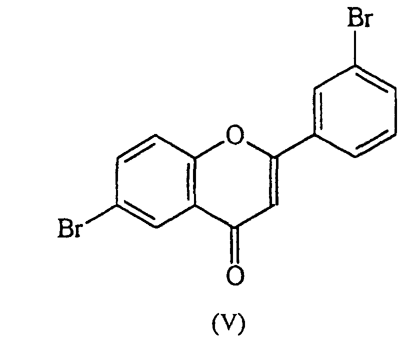

32. Composé de flavonoïde suivant la revendication 26, qui est:

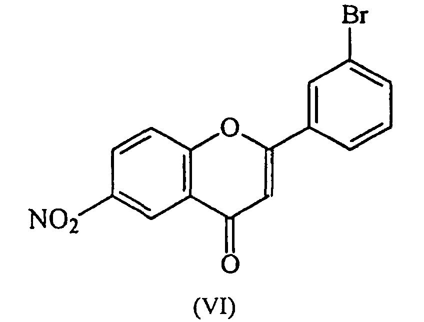

33. Utilisation d'un mélange de composés choisis parmi les composés III, IV, V et VI et/ou de dimères de bi-flavonoïdes de ceux-ci pour la préparation d'un médicament pour le traitement de l'anxiété chez un patient.

34. Utilisation d'un mélange de composés suivant la revendication 33, dans laquelle lesdits composés sont choisis parmi les composés V et VI et/ou des dimères de bi-flavonoïdes de ceux-ci.
